(19) Europäisches Patentamt
European Patent Office
Office européen des brevets

(11) Veröffentlichungsnummer : 0 208 281 B1

(12) # EUROPÄISCHE PATENTSCHRIFT

(45) Veröffentlichungstag der Patentschrift : **30.01.91 Patentblatt 91/05**

(51) Int. Cl.[5] : **C07D 401/06**, A01N 43/50, A01N 43/52, A01N 43/647, A01N 43/56

(21) Anmeldenummer : **86109188.2**

(22) Anmeldetag : **04.07.86**

(54) **Chinolin-8-carbonsäureazolide, ein Verfahren zu ihrer Herstellung und ihre Verwendung als Herbizide.**

Die Akte enthält technische Angaben, die nach dem Eingang der Anmeldung eingereicht wurden und die nicht in dieser Patentschrift enthalten sind.

(30) Priorität : **12.07.85 DE 3524918**

(43) Veröffentlichungstag der Anmeldung : **14.01.87 Patentblatt 87/03**

(45) Bekanntmachung des Hinweises auf die Patenterteilung : **30.01.91 Patentblatt 91/05**

(84) Benannte Vertragsstaaten : **AT BE CH DE FR GB IT LI NL SE**

(56) Entgegenhaltungen : **EP-A- 0 060 429**

(73) Patentinhaber : **BASF Aktiengesellschaft**
**Carl-Bosch-Strasse 38**
**D-6700 Ludwigshafen (DE)**

(72) Erfinder : **Plath, Peter, Dr.**
**Hans-Balcke-Strasse 13**
**D-6710 Frankenthal (DE)**
Erfinder : **Eicken, Karl, Dr.**
**Am Hüttenwingert 12**
**D-6706 Wachenheim (DE)**
Erfinder : **Hagen, Helmut, Dr.**
**Max-Slevogt-Strasse 17e**
**D-6710 Frankenthal (DE)**
Erfinder : **Kohler, Rolf-Dieter, Dr.**
**Amselweg 3**
**D-6803 Edingen-Neckarhausen (DE)**
Erfinder : **Markert, Jürgen, Dr.**
**Am Speyerweg 26**
**D-6704 Mutterstadt (DE)**
Erfinder : **Meyer, Norbert, Dr.**
**Dossenheimer Weg 22**
**D-6802 Ladenburg (DE)**
Erfinder : **Würzer, Bruno, Dr.**
**Rüdigerstrasse 13**
**D-6701 Otterstadt (DE)**

EP 0 208 281 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft neue Chinolin-8-carbonsäureazolide, ein Verfahren zu ihrer Herstellung, Herbizide, die diese Verbindungen enthalten, sowie ein Verfahren zur Bekämpfung von unerwünschtem Pflanzenwuchs.

Herbizide Mittel auf Basis von Dichlor-Chinolinderivaten, die in 8-Stellung substituiert sind, sind aus EP-A 60 429 und EP-A 106 949 bekannt.

In der AT-A-359 497 ist ein Verfahren zur Herstellung von 8-(Tetrazol-5-yl-carbamoyl)-Chinolinderivaten beschrieben. Die so herstellbaren Chinolinderivate können als Antiallergika verwendet werden.

Es wurden nun neue Chinolin-8-carbonsäureazolide der Formel I

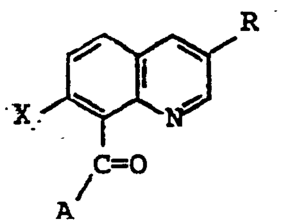

(I),

in der

R für Wasserstoff, Halogen, $C_1$-$C_4$-Alkyl, $C_1$-$C_4$-Halogenalkyl, Formyl oder Cyano steht,

X Halogen und

A einen fünfgliedrigen Heterocyclus mit zwei oder drei Stickstoffatomen im Ring bedeuten, der über ein Stickstoffatom an die Carbonylgruppe geknüpft ist, und der gegebenenfalls durch Halogen, $C_1$-$C_4$-Alkyl, $C_1$-$C_4$-Alkoxycarbonyl, Cyano, Trifluormethyl, Mercapto, Methylthio, Methylsulfonyl, Methoxy, Amino oder Nitro ein- oder mehrfach substituiert sein kann, und der weiterhin für den Fall, daß es sich um in 2-Stellung gegebenenfalls durch Methyl oder Trifluormethyl substituiertes Imidazol oder 1,2,3-Triazol handelt, auch benzoannelliert sein gefunden.

Die neuen Azolide zeigen gute herbizide Wirkung bei gleichzeitiger Verträglichkeit für bestimmte Kulturpflanzen. Außerdem weisen sie bezüglich der Mobilität im Boden günstige Eigenschaften auf.

In der Formel I steht

R z.B. für Wasserstoff, Fluor, Chlor, Brom, Methyl, Ethyl, n-Propyl, Isopropyl, sec-Butyl oder Isobutyl. Bevorzugte Reste $R^1$ sind Chlor oder Methyl.

X kann z.B. Fluor, Chlor oder Brom, vorzugsweise aber Chlor sein.

A bedeutet z.B. unsubstituiertes Pyrazol oder einfach bis dreifach substituiertes Pyrazol, wie 4-Chlorpyrazol, 4-Nitropyrazol, 3,5-Dimethylpyrazol, 3-Methyl-5-chlor-pyrazol, 3,5-Dimethyl-4-chlor-pyrazol, 3,5-Dimethyl-4-brompyrazol, 3,5-Dimethyl-4-nitropyrazol, 3-Trifluormethylpyrazol, 3-Trifluormethyl-5-chlor-pyrazol, 3,5-Bis--trifluormethyl-pyrazol, 4-Methoxycarbonyl-pyrazol oder 4-Cyano-pyrazol.

A bedeutet weiterhin z.B. unsubstituiertes Imidazol, einfach bis dreifach substituiertes Imidazol, wie 4-Chlor-imidazol, 2,4-Dichlor-imidazol, 4,5-Dichlorimidazol, 4-Nitroimidazol, 4-Nitro-5-chlor-imidazol, 4-Nitro-5-methylthio-imidazol oder 4-Nitro-5-methylsulfonyl-imidazol.

A bedeutet weiterhin Benzimidazol oder einfach substituiertes Benzimidazol, wie 2-Trifluormethylbenzimidazol oder 2-Methylbenzimidazol.

A steht auch für unsubstituiertes Triazol, z.B. 1,2,4-Triazol, 1,2,3-Triazol, oder für ein- oder zweifach substituiertes Triazol, beispielsweise 5-Chlor-1,2,4-triazol, 3-(5)-Methyl-1,2,4-triazol, 3,5-Dimethyl-1,2,4-triazol, 3-Methyl-5-chlor-1,2,4-triazol, 3,5-Dichlor-1,2,4-triazol, 3,5-Bis-trifluormethyl-1,2,4-triazol, 5-Amino-1,2,4-triazol, 5-Amino-3-methyl-1,2,4-triazol, 5-Amino-3-n-butyl-1,2,4-triazol, 5-Mercapto-1,2,4-triazol, 5-Methoxy-1,2,4-triazol oder 5-Methylthio-1,2,4-triazol sowie für 1,2,3-Benztriazol.

Bevorzugte Azole sind 1,2,4-Triazol, Imidazol, 5-Mercapto-1,2,4-triazol und 5-Amino-1,2,4-triazol.

Die erfindungsgemäßen Chinolin-8-carbonsäureazolide werden erhalten durch Umsetzung eines Säurechlorids der Formel II

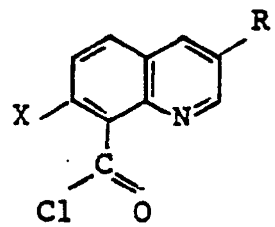

(II)

mit einem Azol der Formel A-H, wobei R, X und A jeweils die obengenannte Bedeutung besitzen. Das Säurechlorid kann dabei auch in Form seines Hydrochlorids eingesetzt werden.

Bedingt durch die bei Azolen der Formel A-H auftretenden tautomeren Formen (z.B. 4-Chlor-1H-imidazol und 4-Chlor-3H-imidazol) können bei der Umsetzung gegebenenfalls auch isomere Azolide entstehen.

Die Umsetzung der Chinolin-8-carbonsäurechloride mit den Azolen findet in Gegenwart einer Base und eines inerten Lösungsmittels bei einer Temperatur von 0 bis 100°C, vorzugsweise 20 bis 60°C statt.

Pro Mol Säurechlorid werden 2 bis 10 Mol Base zugesetzt. Als Basen eignen sich tertiäre Amine, wie Triethylamin, Tri-n-propylamin, N,N-Dimethylcyclohexylamin, N-Methylpiperidin oder Pyridin. Die Verwendung von Triethylamin ist bevorzugt.

Geeignete inerte Lösungsmittel sind beispielsweise Methylenchlorid, 1,1,1-Trichlorethan, Tetrahydrofuran, Dioxan, Essigsäureethylester, Toluol oder Chlorbenzol. Die Verwendung von Methylenchlorid und 1,1,1-Trichlorethan ist bevorzugt.

Bei der Durchführung des erfindungsgemäßen Verfahrens werden zweckmäßig das Säurechlorid zusammen mit dem inerten Lösungsmittel und der Base vorgelegt. Dann gibt man bei einer Temperatur von 0 bis 30°C das jeweilige Azol zu und rührt 5 bis 16 Stunden bei einer Temperatur von 20 bis 100°C nach. Das entstandene Azolid, das im allgemeinen schwerlöslich ist, wird anschließend abgesaugt, mit Wasser gewaschen und getrocknet.

Die für die Umsetzung benötigten Chinolin-8-carbonsäurechloride werden erhalten, indem man in an sich bekannter Weise die entsprechenden freien Chinolin-8-carbonsäuren mit Thionylchlorid in einem inerten Lösungsmittel (z.B. Toluol, Xylol oder Chlorbenzol) bei einer Temperatur von 30 bis 120°C umsetzt. Zweckmäßig verwendet man dabei 1,2 bis 20 Mol Thionylchlorid pro Mol freier Säure. Nach Beendigung der Reaktion, die im allgemeinen 3 bis 12 Stunden in Anspruch nimmt, wird das Säurechlorid abgesaugt, gewaschen und getrocknet.

Die jeweiligen Chinolin-8-carbonsäurechloride fallen dabei entweder in freier Form oder als Hydrochlorid an. Für die nachfolgende Azolidbildung ist dies jedoch ohne Bedeutung, da, wie oben ausgeführt, in dieser Stufe sowohl die freien Säurechloride wie auch ihre Hydrochloride verwendet werden können.

Die der Reaktion zugrundeliegenden entsprechenden freien Chinolin-8-carbon säuren sind in der DE-A-3 108 873 beschrieben.

Die folgenden Beispiele sollen die Erfindung näher erläutern.

<u>Beispiel 1</u>

3,7-Dichlorchinolin-8-carbonsäurechlorid

242 g (1 Mol) 3,7-Dichlorchinolin-8-carbonsäure wurden in 1 l Toluol suspendiert, mit 3 ml Dimethylformamid versetzt und auf 60°C erwärmt. Dann tropfte man 238 g (2 Mol) Thionylchlorid so zu, daß eine Temperatur von 60 bis 70°C eingehalten werden konnte. Anschließend wurde zum Sieden erhitzt, bis keine Abgasentwicklung (HCl, $SO_2$) mehr festzustellen war.

Beim Abkühlen fiel das Zielprodukt größtenteils aus. Es wurde durch Absaugen isoliert und mit Diethylether nachgewaschen. Aus dem Filtrat wurde nach Abziehen des Toluols und unverbrauchten Thionylchlorids durch Anreiben mit Diethylether zusätzliches Produkt gewonnen. Man erhält 246 g (94 % d.Th.) eines farblosen Feststoffs mit Fp. 120-122°C.

<u>Beispiel 2</u>

1,2,4-Triazolid von 3,7-Dichlorchinolin-8-carbonsäure

296,5 g (1,1 Mol) des in Beispiel 1 hergestellten Säurechlorids wurden in einer Mischung aus 2,5 l Methylenchlorid und 499 g (4,8 Mol) Triethylamin vorgelegt. Anschließend gab man portionsweise bei 20

bis 30°C 111 g (1,6 Mol) 1,2,4-Triazol zu und ließ 16 Stunden bei 25°C nachrühren. Das Zielprodukt fiel als schwerlöslicher farbloser Feststoff neben Triethylaminhydrochlorid an. Das Feststoffgemisch wurde durch Absaugen vom Methylenchlorid befreit, in 2 l Wasser gegeben, kurz aufgerührt und dann erneut abgesaugt. Nach Waschen mit Wasser wurde bei 50°C unter vermindertem Druck getrocknet. Man erhielt 245 g (76 % d. Th.) eines farblosen Feststoffs mit Fp. 261-263°C (Verbindung Nr. 1).

Analog Beispiel 2 wurden die in Tabelle 1 aufgeführten Verbindungen hergestellt (Bezeichnung der Reste vgl. Formel I, wobei X für Chlor steht) :

(Wie oben dargelegt, können, bedingt durch die bei den Azolen auftretenden tautomeren Formen, isomere Azolide entstehen. Aus diesem Grund wurde auf die genaue Angabe der Verknüpfungsposition am Azolring jeweils verzichtet.)

Tabelle 1

| Verbindung Nr. | R | A | Fp °C |
|---|---|---|---|
| 2 | Cl | Pyrazolyl | 213-215 |
| 3 | Cl | 4-Chlorpyrazolyl | 214-215 |
| 4 | Cl | Imidazolyl | 184-186 |
| 5 | Cl | 5-Nitroimidazolyl | 245 |
| 6 | Cl | Benzimidazolyl | 144-146 |
| 7 | Cl | 3,5-Dimethyl-1,2,4-triazolyl | 210-212 |
| 8 | Cl | 3,(5)-Chlor-1,2,4-triazolyl | 200-201 |
| 9 | Cl | 3,(5)-Amino-1,2,4-triazolyl | 248-249 |
| 10 | Cl | 3,(5)-Mercapto-1,2,4-triazolyl | 120 (zers.) |
| 11 | Cl | 5,(3)-Methylthio-3,(5)-amino-1,2,4--triazolyl | 284-285 |
| 12 | Cl | 5,(3)-n-Butyl-3,(5)-amino-1,2,4--triazolyl | 285-286 |
| 13 | Cl | 1,2,3-Benztriazolyl | 225-227 |
| 14 | $CH_3$ | Pyrazolyl | 206-208 |
| 15 | $CH_3$ | Imidazolyl | 164-166 |
| 16 | $CH_3$ | 4,5-Dichlorimidazolyl | 160-162 |
| 17 | $CH_3$ | Benzimidazolyl | 142-144 |
| 18 | $CH_3$ | 1,2,4-Triazolyl | 233-235 |
| 19 | $CH_3$ | 3,(5)-Mercapto-1,2,4-triazolyl | 300 (zers.) |
| 20 | $CH_3$ | 3,(5)-Amino-1,2,4-triazolyl | 300 |
| 21 | $CH_3$ | Benztriazolyl | 139-141 |

Erfindungsgemäße Verbindungen der Formel I sind weiterhin die in Tabelle 2 genannten Stoffe :

Tabelle 2

| R | X | A | R | X | A |
|---|---|---|---|---|---|
| H | Cl | 1,2,4-Triazolyl | Cl | Cl | 2,4-Dichlorimidazolyl |
| Br | Cl | 1,2,4-Triazolyl | Cl | Cl | 4,5-Dichlorimidazolyl |
| $C_2H_5$ | Cl | 1,2,4-Triazolyl | $CH_3$ | Cl | 4-Nitroimidazolyl |
| i-$C_3H_7$ | Cl | 1,2,4-Triazolyl | $CH_3$ | Cl | 4-Nitro-5-chlor-imidazolyl |
| sec-$C_4H_9$ | Cl | 1,2,4-Triazolyl | Cl | Cl | 4-Nitro-5-S-$CH_3$--imidazolyl |
| $CF_3$ | Cl | 1,2,4-Triazolyl | Cl | Cl | 4-$NO_2$-5-$SO_2CH_3$--imidazolyl |
| Cl | Cl | 4-Nitropyrazolyl | Cl | Cl | 2-$CF_3$-benzimidazolyl |
| Cl | Cl | 3,5-Dimethyl-pyrazolyl | Cl | Cl | 2-$CH_3$-benzimidazolyl |
| Cl | Cl | 3-Methyl-5-chlor-pyrazolyl | $CH_3$ | Cl | 1,2,3-Triazolyl |
| Cl | Cl | 3,5-Dimethyl-4-chlorpyrazolyl | Cl | Cl | 1,2,3-Triazolyl |
| Cl | Cl | 3,5-Dimethyl-4-$NO_2$-pyrazolyl | $CH_3$ | Cl | 5-Chlor-1,2,4--triazolyl |
| $CH_3$ | Cl | 3-Trifluormethyl-pyrazolyl | $CH_3$ | Cl | 3,5-Dimethyl-1,2,4--triazolyl |
| $CH_3$ | Cl | 3-$CF_3$-5-Cl-pyrazolyl | Cl | Cl | 3-Methyl-5-Cl-1,2,4--triazolyl |
| $CH_3$ | Cl | 3,5-$(CF_3)_2$-pyrazolyl | $CH_3$ | Cl | 3,5-$Cl_2$-1,2,4-triazolyl |
| Cl | Cl | 4-$CO_2$-$CH_3$-pyrazolyl | Cl | Cl | 3,5-$(CF_3)_2$-1,2,4--triazolyl |
| Cl | Cl | 4 CN-Pyrazolyl | Cl | Cl | 5-$NH_2$-3-$CH_3$-1,2,4--triazolyl |
| Cl | Cl | 4-Chlorimidazolyl | Cl | Cl | 5-$SCH_3$-1,2,4-triazolyl |
| Cl | F | 1,2,4-Triazolyl | Cl | Cl | 5-$OCH_3$-1,2,4-triazolyl |
| Cl | Br | 1,2,4-Triazolyl | | | |

Die erfindungsgemäßen Azolide besitzen eine überraschende Stabilität gegenüber Wasser und Alkoholen. So ist es beispielsweise möglich, das Imidazolid (Verbindung Nr. 4) aus Methanol umzukristallisieren, ohne daß dabei der betreffende Chinolin-8-carbonsäuremethylester gebildet wird.

Die Hydrolysebeständigkeit wird durch folgendes Beispiel belegt.

Beispiel 3

0,1 g 3,7-Dichlorchinolin-8-carbonsäure-triazolid (Verbindung Nr. 1) wurden mit 85 g lehmiger Sanderde und 100 ml Wasser vermischt und 14 Tage bei 25°C rühren gelassen. Im Abstand von 3 Tagen wurde eine Probe entnommen und dünnschichtchromatographisch untersucht. Dabei zeigte sich, daß unverändert Verbindung Nr. 1 vorlag. Die entsprechende freie Säure konnte nicht nachgewiesen werden.

Die Chinolin-8-carbonsäureazolide der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron, stark polare Lösungsmittel, wie N,N-Dimethylformamid, Dimethylsulfoxid, N-Methylpyrrolidon, Wasser, in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile der Verbindung Nr. 1 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteile Calciumsalz der Dodecylbenzolsulfonsäure und 5 Gewichtsteile des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 9 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 4 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöls besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 15 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 19 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 4 werden mit 12 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensate s und 68 Teile eines paraffinischen Mineralöls innig vermischt. Man erhält eine stabile ölige Dispersion.

Die Applikation der neuen Verbindungen als herbizide Mittel kann im Vorauflaufverfahren und bei Nachauflaufverfahren erfolgen. Bei besonders empfindlichen Kulturpflanzen können Ausbringungstechniken angewandt werden, bei welchen die herbiziden Mittel mit Hilfe von Geräten so gespritzt werden, daß die Blätter der besonders empfindlichen Kulturpflanzen nach Möglichkeit nicht getroffen werden, während die Wirkstoffe auf die Blätter darunterwachsender unerwünschter Pflanzen oder auf die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium derselben ungefähr 0,1 bis 5 kg Wirkstoff/ha und mehr. Im folgenden sei mit einigen Verbindungen beispielhaft gezeigt, welche Wirksamkeit und Selektivität sie haben.

Der Einfluß der neuen Chinolin-8-carbonsäureazolide auf das Wachstum von erwünschten und unerwünschten Pflanzen wird anhand von Gewächshausversuchen aufgezeigt :

Als Kulturgefäße dienen Plastikblumentöpfe mit 300 $cm^3$ Inhalt und lehmigem Sand mit etwa 3,0 % Humus als Substrat. Die Samen der Testpflanzen wurden nach Arten getrennt flach eingesät. Unmittelbar danach erfolgt bei Vorauflaufbehandlung das Aufbringen der Wirkstoffe auf die Erdoberfläche. Sie wurden hierzu in Wasser als Verteilungsmittel suspendiert oder emulgiert und mittels fein verteilender Düsen gespritzt. Die Aufwandmengen betrugen im Vorauflauf 1,0 und 3,0 kg Wirkstoff/ha.

Nach dem Aufbringen der Mittel beregnete man die Gefäße leicht, um Keimung und Wachstum in Gang zu bringen. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern diese nicht durch die Wirkstoffe beeinträchtigt wurden.

Für die Nachauflaufbehandlung zog man die Testpflanzen je nach Wuchsform erst bis zu einer Wuchshöhe von 3 bis 15 cm an und behandelte sie danach. Zur Nachauflaufbehandlung wurden entweder direkt gesäte und in den gleichen Gefäßen aufgewachsene Pflanzen so gewählt oder aber sie wurden erst als Keimpflanzen getrennt angezogen und einige Tage vor der Behandlung in die Versuchsgefäße verpflanzt. Die Aufwandmenge für die Nachauflaufbehandlung betrug 0,5, 1,0 und 3,0 kg Wirkstoff/ha.

In die Versuche waren folgende Testpflanzen einbezogen :

| Botanischer Name | Deutscher Name |
|---|---|
| Avena sativa | Hafer |
| Beta vulgaris | Zuckerrübe |
| Echinochloa crus-galli | Hühnerhirse |
| Galium aparine | Klettenlabkraut |
| Lolium multiflorum | Ital. Raygras |
| Oryza sativa | Reis |
| Sesbania exaltata | Turibaum |
| Sinapis alba | Weißer Senf |
| Triticum aestivum | Weizen |
| Veronica spp. | Ehrenpreisarten |

Die Versuchsgefäße wurden im Gewächshaus aufgestellt, wobei für wärmeliebende Arten wärmere Bereiche (20 bis 35°C) und für solche gemäßigter Klimate 10 bis 20°C bevorzugt wurden. Die Vergleichsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Aufgang und 100 kein Aufgang bzw. völlige Zerstörung

zumindest der oberirdischen Sproßteile.

Bei der Vorauflaufanwendung von 3,0 kg Wirkstoff/ha zeigt beispielsweise Verbindung Nr. 9 gegen Vertreter breitblättiger und grasartiger Pflanzen beachtliche herbizide Aktivität. Mit den beispielhaft ausgewählten Verbindungen Nr. 1 und 9 läßt sich Echinochloa crus galli gut bekämpfen, während die Kulturpflanze Reis allenfalls unwesentliche Schäden erleidet.

Die Wirksamkeit der neuen Verbindungen im Nachauflaufverfahren wird beispielsweise mittels den Verbindungen Nr. 9, 4 und 1 gezeigt, die grasartige und breitblättrige Beispielpflanzen mit einer Aufwandmenge von 3,0 kg Wirkstoff/ha gut bekämpfen.

Gegen unerwünschten breitblättrigen Pflanzenwuchs in Getreidekulturen kann man z.B. die Verbindungen Nr. 15, 19 und 2 mit einer Aufwandmenge von 3 kg Wirkstoff/ha oder die Verbindungen Nr. 15 und 19 mit einer Aufwandmenge von 1,0 kg Wirkstoff/ha im Nachauflaufverfahren einsetzen. Die Schädigung von Hafer und Weizen kann dabei vernachlässigt werden.

Die beispielhaft ausgewählten Verbindungen Nr. 15 und 19 können darüber hinaus auch in Zuckerrüben zur Bekämpfung wichtiger Schadpflanzen verwendet werden.

Weiterhin eignet sich z.B. Verbindung Nr. 1 bei Nachauflaufanwendung mit 0,5 kg Wirkstoff/ha zur Bekämpfung breitblättriger unerwünschter Vegetation in Reis.

In Anbetracht der guten Verträglichkeit für zahlreiche breitblättrige und andere Kulturen und der Vielseitigkeit der Applikationsmethoden können die erfindungsgemäßen Herbizide oder diese enthaltende Mittel noch in einer weiteren großen Zahl von Kulturpflanzen zur Beseitigung unerwünschten Pflanzenwuchses eingesetzt werden.

In Betracht kommen beispielsweise folgende Kulturpflanzen :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |

| Botanischer Name | Deutscher Name |
|---|---|
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Panicum miliaceium | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |

| Botanischer Name | Deutscher Name |
|---|---|
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais (post directed) |

Zur Verbreiterung des Wirkungsspektrums und zur Erzielung auch synergistischer Effekte können die neuen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden.

## Ansprüche

**Ansprüche für die Vertragsstaaten : BE, CH, DE, FR, GB, IT, LI, NL, SE**

1. Chinolin-8-carbonsäureazolide der Formel I

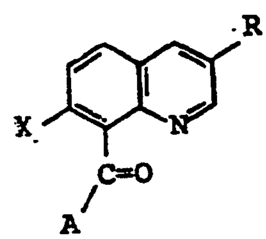

(I),

in der
R für Wasserstoff, Halogen, $C_1$-$C_4$-Alkyl, $C_1$-$C_4$-Halogenalkyl, Formyl oder Cyano steht,
X Halogen und
A einen fünfgliedrigen Heterocyclus mit zwei oder drei Stickstoffatomen im Ring bedeuten, der über ein Stickstoffatom an die Carbonylgruppe geknüpft ist, und der gegebenenfalls durch Halogen, $C_1$-$C_4$-Alkyl, $C_1$-$C_4$-Alkoxycarbonyl, Cyano, Trifluormethyl, Mercapto, Methylthio, Methylsulfonyl, Methoxy, Amino oder Nitro ein- oder mehrfach substituiert sein kann, und der weiterhin für den Fall, daß es sich um in 2-Stellung gegebenenfalls durch Methyl oder Trifluormethyl substituiertes Imidazol oder um 1,2,3-Triazol handelt, auch benzoannelliert sein kann.

2. Chinolin-8-carbonsäureazolide der Formel I nach Anspruch 1, in der

R für Halogen oder $C_1$-$C_4$-Alkyl steht,
X Fluor, Chlor oder Brom ist und
A unsubstituiertes Pyrazol, Imidazol, Benzimidazol oder Triazol ; ein- bis dreifach substituiertes Pyrazol oder Imidazol ; ein- bis zweifach substituiertes Triazol ; oder einfach substituiertes Benzimidazol bedeutet, deren Substituenten Chlor, Nitro, Methyl, Trifluormethyl, Methoxy, Cyano bzw. Methylsulfonyl sein können.

3. Chinolin-8-carbonsäureazolide der Formel I nach Anspruch 1, in der
R für Chlor oder Methyl steht,
X Chlor ist und
A unsubstituiertes Pyrazol, Imidazol, Benzimidazol oder Triazol ; ein- bis dreifach substituiertes Pyrazol oder Imidazol ; ein- bis zweifach substituiertes Triazol ; oder einfach substituiertes Benzimidazol bedeutet, deren Substituenten Chlor, Nitro, Methyl, Trifluormethyl, Methoxy, Cyano bzw. Methylsulfonyl sein können.

4. Chinolin-8-carbonsäureazolid der Formel I nach Anspruch 1, in der
R für Chlor oder Methyl steht,
X Chlor ist und
A 1,2,4-Triazol, Imidazol, 5-Mercapto-1,2,4-triazol oder 5-Amino--1,2,4-triazol bedeutet.

5. Verfahren zur Herstellung von Chinolin-8-carbonsäureazoliden der Formel I gemäß Anspruch 1, dadurch gekennzeichnet, daß man ein Chinolin-8-carbonsäurechlorid der Formel II

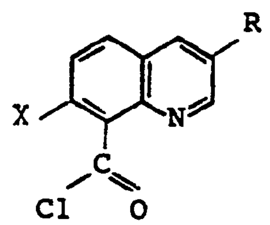

(II)

mit einem Azol der Formel A-H in Gegenwart eines Lösungsmittels und einer Base umsetzt.

6. Herbizid, enthaltend ein Chinolin-8-carbonsäureazolid der Formel I gemäß Anspruch 1 neben hierfür üblichen Trägerstoffen.

7. Herbizid, enthaltend ein Chinolin-8-carbonsäureazolid der Formel I gemäß Anspruch 2 neben hierfür üblichen Trägerstoffen.

8. Herbizid, enthaltend ein Chinolin-8-carbonsäureazolid der Formel I gemäß Anspruch 3 neben hierfür üblichen Trägerstoffen.

9. Herbizid, enthaltend ein Chinolin-8-carbonsäureazolid der Formel I gemäß Anspruch 4 neben hierfür üblichen Trägerstoffen.

10. Verfahren zur Bekämpfung unerwünschten Pflanzenwuchses, dadurch gekennzeichnet, daß man die Pflanzen und/oder die von unerwünschtem Pflanzenwuchs freizuhaltende Fläche mit einem Chinolin-8-carbonsäureazolid gemäß Anspruch 1 behandelt.

**Ansprüche für den Vertragsstaat : AT**

1. Verfahren zur Herstellung von Chinolin-8-carbonsäureazoliden der Formel I

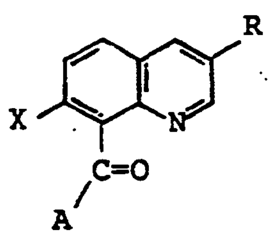

(I),

in der
R für Wasserstoff, Halogen, $C_1$-$C_4$-Alkyl, $C_1$-$C_4$-Halogenalkyl, Formyl oder Cyano steht,
X Halogen und
A einen fünfgliedrigen Heterocyclus mit zwei oder drei Stickstoffatomen im Ring bedeuten, der über ein Stickstoffatom an die Carbonylgruppe geknüpft ist, und der gegebenenfalls durch Halogen, $C_1$-$C_4$-Alkyl, $C_1$-

$C_4$-Alkoxycarbonyl, Cyano, Trifluormethyl, Mercapto, Methylthio, Methylsulfonyl, Methoxy, Amino oder Nitro ein- oder mehrfach substituiert sein kann, und der weiterhin für den Fall, daß es sich um in 2-Stellung gegebenenfalls durch Methyl oder Trifluormethyl substituiertes Imidazol oder um 1,2,3-Triazol handelt, auch benzoannelliert sein kann,
dadurch gekennzeichnet, daß man ein Chinolin-8-carbonsäurechlorid der Formel II

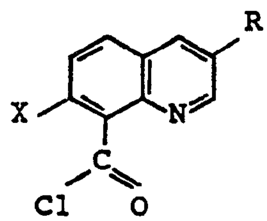

(II)

mit einem Azol der Formel A-H in Gegenwart eines Lösungsmittels und einer Base umsetzt.

2. Herbizid, enthaltend ein Chinolin-8-carbonsäureazolid der Formel I

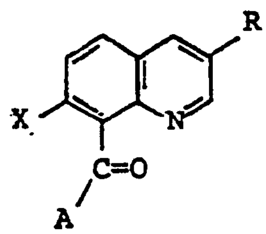

(I),

in der
R für Wasserstoff, Halogen, $C_1$-$C_4$-Alkyl, $C_1$-$C_4$-Halogenalkyl, Formyl oder Cyano steht,
X Halogen und
A einen fünfgliedrigen Heterocyclus mit zwei oder drei Stickstoffatomen im Ring bedeuten, der über ein Stickstoffatom an die Carbonylgruppe geknüpft ist, und der gegebenenfalls durch Halogen, $C_1$-$C_4$-Alkyl, $C_1$-$C_4$-Alkoxycarbonyl, Cyano, Trifluormethyl, Mercapto, Methylthio, Methylsulfonyl, Methoxy, Amino oder Nitro ein- oder mehrfach substituiert sein kann, und der weiterhin für den Fall, daß es sich um in 2-Stellung gegebenenfalls durch Methyl oder Trifluormethyl substituiertes Imidazol oder um 1,2,3-Triazol handelt, auch benzoannelliert sein kann, neben hierfür üblichen Zusatzstoffen.

3. Herbizid, enthaltend ein Chinolin-8-carbonsäureazolid der Formel I, in der
R für Halogen oder $C_1$-$C_4$-Alkyl steht,
X Fluor, Chlor oder Brom ist und
A unsubstituiertes Pyrazol, Imidazol, Benzimidazol oder Triazol ; ein- bis dreifach substituiertes Pyrazol oder Imidazol ; ein- bis zweifach substituiertes Triazol ; oder einfach substituiertes Benzimidazol bedeutet, deren Substituenten Chlor, Nitro, Methyl, Trifluormethyl, Methoxy, Cyano bzw. Methylsulfonyl sein können, neben hierfür üblichen Zusatzstoffen.

4. Herbizid, enthaltend ein Chinolin-8-carbonsäureazolid der Formel I, in der
R für Chlor oder Methyl steht,
X Chlor ist und
A unsubstituiertes Pyrazol, Imidazol, Benzimidazol oder Triazol ; ein- bis dreifach substituiertes Pyrazol oder Imidazol ; ein- bis zweifach substituiertes Triazol ; oder einfach substituiertes Benzimidazol bedeutet, deren Substituenten Chlor, Nitro, Methyl, Trifluormethyl, Methoxy, Cyano bzw. Methylsulfonyl sein können, neben hierfür üblichen Zusatzstoffen.

5. Herbizid, enthaltend ein Chinolin-8-carbonsäureazolid der Formel I, in der
R für Chlor oder Methyl steht,
X Chlor ist und
A 1,2,4-Triazol, Imidazol, 5-Mercapto-1,2,4-triazol oder 5-Amino-1,2,4-triazol bedeutet, neben hierfür üblichen Zusatzstoffen.

6. Verfahren zur Bekämpfung unerwünschten Pflanzenwuchses, dadurch gekennzeichnet, daß man die

Pflanzen und/oder die von unerwünschtem Pflanzenwuchs freizuhaltende Fläche mit einem Chinolin-8-carbonsäureazolid der Formel I gemäß Anspruch 2 behandelt.

7. Verfahren zur Bekämpfung unerwünschten Pflanzenwuchses, dadurch gekennzeichnet, daß man die Pflanzen und/oder die von unerwünschtem Pflanzenwuchs freizuhaltende Fläche mit einer Verbindung I gemäß Anspruch 3 behandelt.

8. Verfahren zur Bekämpfung unerwünschten Pflanzenwuchses, dadurch gekennzeichnet, daß man die Pflanzen und/oder die von unerwünschtem Pflanzenwuchs freizuhaltende Fläche mit einer Verbindung I gemäß Anspruch 4 behandelt.

9. Verfahren zur Bekämpfung unerwünschten Pflanzenwuchses, dadurch gekennzeichnet, daß man die Pflanzen und/oder die von unerwünschtem Pflanzenwuchs freizuhaltende Fläche mit einem Verbindung I gemäß Anspruch 5 behandelt.

## Claims

### Claims for the Contracting States :BE, CH, DE, FR, GB, IT, LI, NL, SE

1. A quinoline-8-carboxylic acid azolide of the formula I

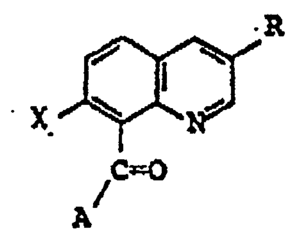

(I)

where R is hydrogen, halogen, $C_1$-$C_4$-alkyl, $C_1$-$C_4$-haloalkyl, formyl or cyano, X is halogen and A is a five-membered heterocyclic structure which contains two or three nitrogen atoms in the ring, is bonded to the carbonyl group via a nitrogen atom and may be unsubstituted or monosubstituted or polysubstituted by halogen, $C_1$-$C_4$-alkyl, $C_1$-$C_4$-alkoxycarbonyl, cyano, trifluoromethyl, mercapto, methylthio, methylsulfonyl, methoxy, amino or nitro, and, when the heterocyclic structure is 1,2,3-triazole or imidazole which is unsubstituted or substituted in the 2-position by methyl or trifluoromethyl, may furthermore be benzofused.

2. A quinoline-8-carboxylic acid azolide of the formula I as claimed in claim 1, where R is halogen or $C_1$-$C_4$-alkyl, X is fluorine, chlorine or bromine and A is unsubstituted pyrazole, imidazole, benzimidazole or triazole ; mono-, di- or trisubstituted pyrazole or imidazole ; mono- or disubstituted triazole ; or monosubstituted benzimidazole, the substituents of which may be chlorine, nitro, methyl, trifluoromethyl, methoxy, cyano or methylsulfonyl.

3. A quinoline-8-carboxylic acid azolide of the formula I as claimed in claim 1, where R is chlorine or methyl, X is chlorine and A is unsubstituted pyrazole, imidazole, benzimidazole or triazole ; mono-, di- or trisubstituted pyrazole or imidazole ; mono- or disubstituted triazole ; or monosubstituted benzimidazole, the substituents of which may be chlorine, nitro, methyl, trifluoromethyl, methoxy, cyano or methylsulfonyl.

4. A quinoline-8-carboxylic acid azolide of the formula I as claimed in claim 1, where R is chlorine or methyl, x is chlorine and A is 1,2,4-triazole, imidazole, 5-mercapto-1,2,4-triazole or 5-amino-1,2,4-triazole.

5. A process for preparing a quinoline-8-carboxylic acid azolide of the formula I as claimed in claim 1, wherein a quinoline-8-carbonyl chloride of the formula II

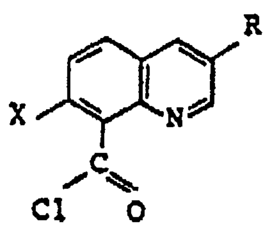

(II)

is reacted with an azole of the formula A-H in the presence of a solvent and a base.

6. A herbicide containing a quinoline-8-carboxylic acid azolide of the formula I as claimed in claim 1,

and conventional carriers therefor.

7. A herbicide containing a quinoline-8-carboxylic acid azolide of the formula I as claimed in claim 2, and conventional carriers therefor.

8. A herbicide containing a quinoline-8-carboxylic acid azolide of the formula I as claimed in claim 3, and conventional carriers therefor.

9. A herbicide containing a quinoline-8-carboxylic acid azolide of the formula I as claimed in claim 4, and conventional carriers therefor.

10. A process for controlling undesirable plant growth, wherein the plants and/or the area to be kept free from undesirable plant growth are or is treated with a quinoline-8-carboxylic acid azolide as claimed in claim 1.

**Claims for the Contracting State : AT**

1. A process for preparing a quinoline-8-carboxylic acid azolide of the formula I

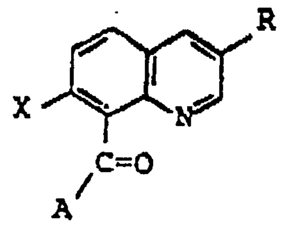

(I)

where R is hydrogen, halogen, $C_1$-$C_4$-alkyl, $C_1$-$C_4$-haloalkyl, formyl or cyano, X is halogen and A is a five-membered heterocyclic structure which contains two or three nitrogen atoms in the ring, is bonded to the carbonyl group via a nitrogen atom and may be unsubstituted or monosubstituted or polysubstituted by halogen, $C_1$-$C_4$-alkyl, $C_1$-$C_4$-alkoxycarbonyl, cyano, trifluoromethyl, mercapto, methylthio, methylsulfonyl, methoxy, amino or nitro, and, when the heterocyclic structure is 1,2,3-triazole or imidazole which is unsubstituted or substituted in the 2-position by methyl or trifluoromethyl, may furthermore be benzofused, wherein a quinoline-8-carbonyl chloride of the formula II

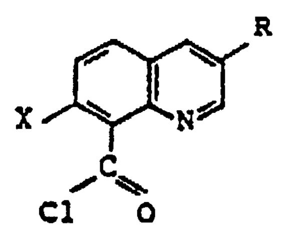

(II)

is reacted with an azole of the formula A-H in the presence of a solvent and a base.

2. A herbicide containing a quinoline-8-carboxylic acid azolide of the formula I

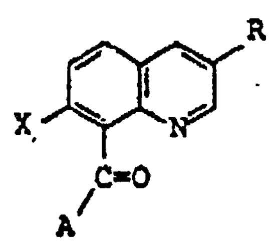

(I)

where R is hydrogen, halogen, $C_1$-$C_4$-alkyl, $C_1$-$C_4$-haloalkyl, formyl or cyano, X is halogen and A is a five-membered heterocyclic structure which contains two or three nitrogen atoms in the ring, is bonded to the carbonyl group via a nitrogen atom and may be unsubstituted or monosubstituted or polysubstituted by halogen, $C_1$-$C_4$-alkyl, $C_1$-$C_4$-alkoxycarbonyl, cyano, trifluoromethyl, mercapto, methylthio, methylsulfonyl, methoxy, amino or nitro, and, when the heterocyclic structure is 1,2,3-triazole or imidazole which is unsubstituted or substituted in the 2-position by methyl or trifluoromethyl, may furthermore be benzofused, and

conventional additives therefor.

3. A herbicide containing a quinoline-8-carboxylic acid azolide of the formula I, where R is halogen or $C_1$-$C_4$-alkyl, X is fluorine, chlorine or bromine and A is unsubstituted pyrazole, imidazole, benzimidazole or triazole ; mono-, di- or trisubstituted pyrazole or imidazole ; mono- or disubstituted triazole ; or monosubstituted benzimidazole, the substituents of which may be chlorine, nitro, methyl, trifluoromethyl, methoxy, cyano or methylsulfonyl, and conventional additives therefor.

4. A herbicide containing a quinoline-8-carboxylic acid azolide of the formula I, where R is chlorine or methyl, X is chlorine and A is unsubstituted pyrazole, imidazole, benzimidazole or triazole ; mono-, di- or trisubstituted pyrazole or imidazole ; mono- or disubstituted triazole ; or monosubstituted benzimidazole, the substituents of which may be chlorine, nitro, methyl, trifluoromethyl, methoxy, cyano or methylsulfonyl, and conventional additives therefor.

5. A herbicide containing a quinoline-8-carboxylic acid azolide of the formula I, where R is chlorine or methyl, X is chlorine and A is 1,2,4-triazole, imidazole, 5-mercapto-1,2,4-triazole or 5-amino-1,2,4-triazole, and conventional additives therefor.

6. A process for controlling undesirable plant growth, wherein the plants and/or the area to be kept free from undesirable plant growth are or is treated with a quinoline-8-carboxylic acid azolide of the formula I as claimed in claim 2.

7. A process for controlling the growth of undesirable plant growth, wherein the plants and/or the area to be kept free from undesirable plant growth are or is treated with a compound I as claimed in claim 3.

8. A process for controlling undesirable plant growth, wherein the plants and/or the area to be kept free from undesirable plant growth are or is treated with a compound I as claimed in claim 4.

9. A process for controlling undesirable plant growth, wherein the plants and/or the area to be kept free from undesirable plant growth are or is treated with a compound I as claimed in claim 5.

## Revendications

### Revendications pour les Etats contractant : BE, CH, DE, FR, GB, IT, LI, NL, SE

1. Azolides d'acide quinoline-8-carboxylique de la formule I

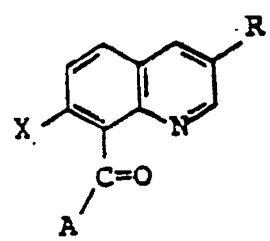

(I),

dans laquelle
R représente hydrogène, halogène, alkyle en $C_1$-$C_4$, halogénoalkyle en $C_1$-$C_4$, formyle ou cyano,
X halogène et
A un hétérocycle à cinq chaînons ayant deux ou trois atomes d'azote dans le noyau qui est lié au groupe carboxyle par l 'intermédiaire d'un atome d'azote, et qui peut éventuellement être substitué une ou plusieurs fois par halogène, alkyle en $C_1$-$C_4$, alcoxycarbonyle en $C_1$-$C_4$, cyano, trifluorométhyle, mercapto, méthylthio, méthylsulfonyle, méthoxy, amino ou nitro et qui peut, en outre, dans le cas où il s'agit d'imidazole substitué éventuellement, en position 2, par méthyle ou trifluorométhyle, ou de 1,2-3-triazole, être aussi benzocondensé.

2. Azolides d'acide quinoline-8-carboxylique de la formule I, selon la revendication 1, dans laquelle
R représente halogène ou alkyle en $C_1$-$C_4$
X est fluor, chlore ou brome et
A représente pyrazole, imidazole, benzimidazole ou triazole, non substitués, pyrazole ou imidazole substitués une à trois fois, triazole substitué une à deux fois, ou un benzimidazole substitué une fois, dont les substituants peuvent être chlore, nitro, méthyle, trifluorométhyle, méthoxy, cyano ou méthylsulfonyle.

3. Azolides d'acide quinoline-8-carboxylique de la formule I, selon la revendication 1 dans laquelle
R est mis pour chlore ou méthyle,
X est chlore et
A représente pyrazole, imidazole, benzimidazole ou triazole, non substitués ; pyrazole ou imidazole subs-

tués une à trois fois ; ou un benzimidazole substitué une fois dont les substituants peuvent être chlore, nitro, méthyle, trifluorométhyle, méthoxy, cyano ou méthylsulfonyle.

4. Azolides d'acide quinoline-8-carboxylique de la formule I, selon la revendication 1, dans laquelle
R est mis pour chlore ou méthyle
X est chlore et
A représente 1,2,4-triazole, imidazole, 5-mercapto-1-2,4-triazole ou 5-amino-1,2,4-triazole

5. Procédé de préparation d'azolides d'acide quinoline-8-carboxylique de la formule I selon la revendication 1, caractérisé par le fait qu'on fait réagir un chlorure d'acide quinoline-8-carboxylique de la formule II

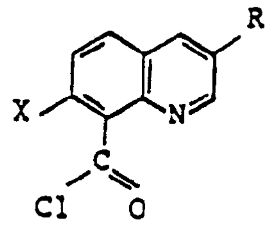

(II)

avec un azole de la formule A-H en présence d'un solvant et d'une base.

6. Herbicide, contenant un azolide d'acide quinoline-8-carboxylique de la formule I, selon la revendication 1, à côté de véhicules usuels pour cela.

7. Herbicide, contenant un azolide d'acide quinoline-8-carboxylique de la formule I, selon la revendication 2, à côté de véhicules usuels pour cela.

8. Herbicide, contenant un azolide d'acide quinoline-8-carboxylique de la formule I, selon la revendication 3, à côté de véhicules usuels pour cela.

9. Herbicide, contenant un azolide d'acide quinoline-8-carboxylique de la formule I, selon la revendication 4, à côté de véhicules usuels pour cela.

10. Procédé de lutte contre la croissance de plantes indésirables, caractérisé par le fait qu'on traite les plantes et/ou les surfaces à maintenir libres d'une corissance de plantes indésirables, avec un azolide d'acide quinoline-8-carboxylique de la formule I, selon la revendication 1.

**Revendications pour l'Etat contractant : AT**

1. Procédé de préparation d'azolides d'acide quinoline-8-carboxylique de la formule I

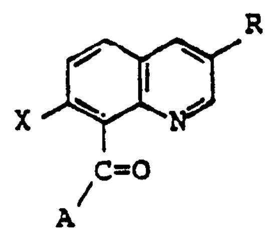

(I),

dans laquelle
R représente hydrogène, halogène, alkyle en $C_1$-$C_4$, halogénoalkyle en $C_1$-$C_4$, formyle ou cyano,
X halogène et
A un hétérocycle à cinq chaînons ayant deux ou trois atomes d'azote dans le noyau qui est lié au groupe carboxyle par l'intermédiaire d'un atome d'azote, et qui peut éventuellement être substitué une ou plusieurs fois par halogène, alkyle en $C_1$-$C_4$, alcoxycarbonyle en $C_1$-$C_4$, cyano, trifluorométhyle, mercapto, méthylthio, méthylsulfonyle, méthoxy, amino ou nitro et qui peut, en outre, dans le cas où il s'agit d'imidazole substitué éventuellement, en position 2, par méthyle ou trifluorométhyle, ou de 1,2-3-triazole, être aussi benzocondensé,

caractérisé par le fait qu'on fait réagir un chlorure d'acide quinoline-8-carboxylique de la formule II

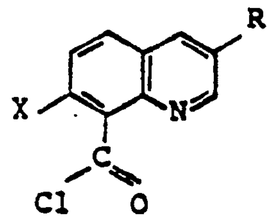

(II)

avec un azole de la formule A-H en présence d'un solvant et d'une base.

2. Herbicide contenant un azolide d'acide quinoline-8-carboxylique de la formule I

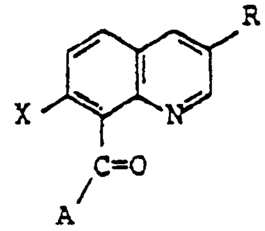

(I),

dans laquelle
R représente hydrogène, halogène, alkyle en $C_1$-$C_4$, halogénoalkyle en $C_1$-$C_4$, formyle ou cyano,
X halogène et
A un hétérocycle à cinq chaînons ayant deux ou trois atomes d'azote dans le noyau qui est lié au groupe carboxyle par l'intermédiaire d'un atome d'azote, et qui peut éventuellement être substitué une ou plusieurs fois par halogène, alkyle en $C_1$-$C_4$, alcoxycarbonyle en $C_1$-$C_4$, cyano, trifluorométhyle, mercapto, méthylthio, méthylsulfonyle, méthoxy, amino ou nitro et qui peut, en outre, dans le cas où il s'agit d'imidazole substitué éventuellement, en position 2, par méthyle ou trifluorométhyle, ou de 1,2-3-triazole, être aussi benzocondensé, à côté d'additifs usuels pour cela.

3. Herbicide contenant un azolide d'acide quinoline-8-carboxylique de la formule I, dans laquelle
R représente halogène ou alkyle en $C_1$-$C_4$
X est fluor, chlore ou brome et
A représente pyrazole, imidazole, benzimidazole ou triazole, non substitués, pyrazole ou imidazole substitués une à trois fois, triazole substitué une à deux fois, ou un benzimidazole substitué une fois, dont les substituants peuvent être chlore, nitro, méthyle, trifluorométhyle, méthoxy, cyano ou méthylsulfonyle, à côté de d'additifs usuels pour cela.

4. Herbicide contenant un azolide d'acide quinoline-8-carboxylique de la formule I, dans laquelle
R est mis pour chlore ou méthyle,
X est chlore et
A représente pyrazole, imidazole, benzimidazole ou triazole, non substitués ; pyrazole ou imidazole substitués une à trois fois ; ou un benzimidazole substitué une fois dont les substituants peuvent être chlore, nitro, méthyle, trifluorométhyle, méthoxy, cyano ou méthylsulfonyle,
à côté de d'additifs usuels pour cela.

5. Herbicide contenant un azolide d'acide quinoline-8carboxylique de la formule I, dans laquelle
R est mis pour chlore ou méthyle
X est chlore et
A représente 1,2,4-triazole, imidazole, 5-mercapto-1-2,4-triazole ou 5-amino-1,2,4-triazole
à côté de d'additifs usuels pour cela.

6. Procédé de lutte contre la croissance des plantes indésirables, caractérisé par le fait qu'on traite les plantes et/ou les surfaces à maintenir libres de la croissance des plantes indésirables avec un azolide d'acide quinoline-8-carboxylique de la formule I selon la revendication 2.

7. Procédé de lutte contre la croissance des plantes indésirables, caractérisé par le fait qu'on traite les plantes et/ou les surfaces à maintenir libres de la croissance des plantes indésirables avec un composé I selon la revendication 3.

8. Procédé de lutte contre la croissance des plantes indésirables, caractérisé par le fait qu'on traite les plantes et/ou les surfaces à maintenir libres de la croissance des plantes indésirables avec un composé I selon la revendication 4.

9. Procédé de lutte contre la croissance des plantes indésirables, caractérisé par le fait qu'on traite les plantes et/ou les surfaces à maintenir libres de la croissance des plantes indésirables avec un composé

selon la revendication 5.